# EUROPEAN PATENT APPLICATION

(11) **EP 2 947 589 A1**
(43) Date of publication of application: **25.11.2015**
(21) Application number: 14305775.0
(22) Date of filing: 23.05.2014
(51) Int. Cl.: G06F 19/22

(54) **Method and apparatus for controlling a decoding of information encoded in synthesized oligos**

(71) Applicant: Thomson Licensing, 92130 Issy-les-Moulineaux (FR)
(72) Inventor: Huetter, Ingo, 30982 Pattensen (DE); Gaedke, Klaus, 30659 Hannover (DE); Chen, Xiaoming, 30165 Hannover (DE); Blawat, Meinolf, 30659 Hannover (DE)
(74) Representative: Rittner, Karsten

(57) **Abstract**

A method for controlling a decoding of information encoded in a plurality of synthesized oligos, encoded using an error detection code identified by an associated identifier, and an apparatus configured to perform the method are described. A sequenced oligo of the synthesized oligos is fetched (11). The identifier associated with the sequenced oligo is read (12). The error detection code of the information encoded in the sequenced oligo is evaluated (13). Information encoded in the oligo is stored (14), if for a first time for an oligo having associated a read identifier the evaluated error detection code indicates no error. The processing is repeated (15) for subsequent oligos until, for at least a defined set of different identifiers of an expected set, information encoded in corresponding sequenced oligos for which the evaluated error detection code indicates no error have been stored.

## Description

### FIELD OF THE INVENTION

The invention relates to a method and an apparatus for controlling a decoding of information encoded in nucleic acid strands, such as DNA strands. In particular, the invention relates to a method and an apparatus for controlling a decoding of portions of information encoded in synthesized oligos, and to a computer readable storage medium.

### BACKGROUND OF THE INVENTION

A nucleic acid is a polymeric macromolecule and consists of a sequence of monomers known as nucleotides. Each nucleotide consists of a sugar component, a phosphate group and a nitrogenous base or nucleobase. Nucleic acid molecules where the sugar component of the nucleotides is deoxyribose are DNA (deoxyribonucleic acid) molecules, whereas nucleic acid molecules where the sugar component of the nucleotides is ribose are referred to as RNA (ribonucleic acid) molecules. DNA and RNA are biopolymers appearing in living organisms.

Nucleic acid molecules are assembled as chains or strands of nucleotides. Nucleic acid molecules can be generated artificially and their chain structure can be used for encoding any kind of user data. For storing data in synthesized, i.e. artificially created, DNA or RNA, usually short DNA or RNA fragments (oligonucleotides, short: oligos) are generated. With these nucleic acid fragments, a data storage system can be realized wherein data are stored in nucleic acid molecules. The synthesized nucleic acid molecules carry the information encoded by the succession of the nucleotides forming the nucleic acid molecules. Each of the synthesized nucleic acid molecules consists of a sequence or chain of nucleotides generated by a bio-chemical process and represents an oligo or nucleic acid fragment wherein the sequence or cascade of the nucleotides encodes a code word sequence corresponding to a portion of information. For example, in a DNA storage system, short DNA fragments are generated. These molecules can be stored and the information can be retrieved by reading the sequence of nucleotides using a sequencer.

In this context, the terms "nucleic acid fragment", "oligonucleotide" and "oligo" are used interchangeably and refer to a short nucleic acid strand. The term "short" in this context is to be understood as short in comparison to a length of natural DNA which encodes genetic instructions used by living organisms and which may consist of millions of nucleotides. Synthesized oligos may contain more than one, for example more than hundred, e.g. between 100 and 300, or several thousands of nucleotides.

Oligonucleotide synthesis or nucleic acid synthesis is the chemical synthesis of oligos with a defined chemical structure, i.e., with a defined sequence of nucleotides, which can be generated by a nucleic acid synthesizer. In other words, a synthesizer can be used to generate artificial, synthetic fragments of nucleic acid molecules, for example DNA fragments, i.e. DNA oligos. This technology enables a provision of data storage systems wherein a write process is based on the creation of nucleic acid fragments as sequences of nucleotides which encode information to be stored.

Digital information storage in synthesized DNA or RNA may provide a high-capacity, low-maintenance information storage. DNA storage has been investigated in "Next-generation digital information storage", Church et al., Science 337, 1628, 2012, and in "Towards practical, high-capacity, low-maintenance information storage in synthesized DNA", Goldman et al., Nature, vol. 494, 2013.

The synthesized nucleic acid fragments can be stored. The information can be retrieved from the stored nucleic acid fragments by sequencing of the nucleic acid fragments, which is a process of determining the order of nucleotides within the particular nucleic acid fragment. For correct sequencing of nucleic acid fragments by a sequencer, the synthesized nucleic acid fragments, i.e., oligos or nucleic acid strands, are first amplified, i.e., replicated multiple times, e.g. by several orders of magnitude, for example using polymerase chain reaction (PCR). The sequencing can be interpreted as a read process. The read out order of nucleotides is processed or decoded to recover the original information stored in the nucleic acid fragment. In a nucleic acid storage system the oligos are synthesized, i.e. nucleic acid strands to be stored are created, amplified, i.e., the number of each single oligo is increased , e.g., to several hundreds or thousands, and sequenced, i.e., the sequence of nucleotides for each oligo is analyzed. In order to recover the stored source information by analyzing the sequenced data, all related sequenced oligos need to be determined.

Each synthesized oligo used for storing digital data usually contains a unique identifier (ID), for example an identification number, address or other coding, for identifying the particular oligo, while at least some of the remaining nucleotides are used for storing the data.

The data can be any kind of sequential digital source data to be stored, e.g., sequences of binary or quaternary code symbols, corresponding to digitally, for example binary, encoded information, such as textual, image, audio or video data.

Due to the limited oligo length, the data is usually distributed to a plurality of oligos each identified with an ID.

As the amplification process and the sequencing introduce errors in the oligos at different locations, for example, less than 50% of the sequenced oligos may contain the correct information. Therefore, as part of the decoding process, the correct nucleotide sequences can be determined by performing majority decisions between oligos with the same given ID.

Each of the originally synthesized oligos is amplified and sequenced and the retrieved information is stored in a memory device until sequencing of all oligos is finished. Due to the large amount of oligos to be sequenced for each ID and the unsorted occurrence of the sequenced oligos, the decoding process can be very time, storage and processing power consuming.

### SUMMARY OF THE INVENTION

It is an object of the present invention to provide a solution for decoding information encoded in synthesized oligos, which allows reliable decoding at a high overall decoding speed, while requiring reduced memory and reduced processing power.

According to an aspect of the invention, a method for controlling a decoding of portions of information encoded in a plurality of synthesized oligos, encoded using an error detection code and identified by an associated identifier, comprises:
- fetching a sequenced oligo of the plurality of synthesized oligos;
- reading the identifier associated with the sequenced oligo;
- evaluating the error detection code of the sequenced oligo;
- storing the portion of information encoded in the sequenced oligo, if for a first time for an oligo having associated the read identifier the evaluated error detection code indicates no error; and
- repeating the fetching, reading, evaluating and storing until, for at least a defined set of different identifiers of an expected set of identifiers, portions of information encoded in corresponding sequenced oligos for which the evaluated error detection code indicates no error have been stored.

Accordingly, an apparatus for controlling a decoding of portions of information encoded in a plurality of synthesized oligos, encoded using an error detection code and identified by an associated identifier, comprises:
- a fetching unit configured to fetch sequenced oligos of the plurality of synthesized oligos;
- a reader unit, connected to the fetching unit and configured to read the identifiers associated with the sequenced oligos;
- an evaluation unit, connected to the fetching unit and configured to evaluate the error detection codes of the portions of information encoded in the sequenced oligos;
- a storage unit, connected to the fetching unit, the reader unit and the evaluation unit, and configured to store portions of information encoded in sequenced oligos, if for a first time for an oligo having associated a read identifier the evaluated error detection code indicates no error; and
- a controller unit, connected to the fetching unit, the reader unit, the evaluation unit and the storage unit, and configured to repeatedly actuate the fetching unit, the reader unit, the evaluation unit and the storage unit until, for at least a defined set of different identifiers of an expected set of identifiers, portions of information encoded in corresponding sequenced oligos for which the evaluated error detection code indicates no error have been stored.

Further, a computer readable storage medium has stored therein instructions enabling control of a decoding of portions of information encoded in a plurality of synthesized oligos, encoded using an error detection code and identified by an associated identifier, which, when executed by a computer, cause the computer to:
- actuate a fetching unit connectable to the computer to fetch a sequenced oligo of the plurality of synthesized oligos;
- read the identifier associated with the sequenced oligo;
- evaluate the error detection code of the sequenced oligo;
- store the portion of information encoded in the sequenced oligo, if for a first time for an oligo having associated the read identifier the evaluated error detection code indicates no error; and
- repeat the actuating the fetching, the reading, the evaluating and the storing until, for at least a defined set of identifiers of an expected set of identifiers, portions of information encoded in corresponding sequenced oligos for which the evaluated error detection code indicates no error have been stored.

The computer readable medium has stored therein instructions which, when executed by a computer, cause the computer to perform steps of the described method.

The term "plurality of synthesized oligos" refers to the collection of oligos artificially created to encode user or source information and comprises the originally synthesized oligos and, if applicable, their copies generated by amplification.

The term "fetching a sequenced oligo of the plurality of synthesized oligos" refers to receiving a result of a sequencing operation performed on an oligo of the plurality of synthesized oligos. In an embodiment "fetching a sequenced oligo" comprises sequencing the oligo and providing the result. Therefore, in one embodiment the sequencing is part of the fetching and is performed during execution of the method, whereas in another embodiment "fetching a sequenced oligo" refers to fetching a sequenced oligo after a sequencing operation has been performed on the plurality of synthesized oligos.

The term "expected set of identifiers" refers to a collection of distinct identifiers and comprises one or more, potentially all, of the identifiers.

In one embodiment a "defined set of different identifiers" refers to a predefined set or amount of different identifiers, for example a set of contiguous IDs or IDs selected according to a rule that may depend, e.g., on the type, encoding scheme or content of the encoded information. In another embodiment the defined set may be changed dynamically during the processing, for example depending on the type, encoding scheme or content of the information processed or stored up to a particular, e.g. the current, moment within the processing. For example, if a particular portion of information has been found, certain of the portions may remain required to be found and decoded while others may then become dispensable.

According to the proposed solution, an error detection code is used to check, whether a fetched sequenced oligo contains correct information. Only new correct portions of information are stored, whereas incorrect or previously already found correct portions of information are not. This processing is repeated for subsequent oligos until all desired portions of information have been found. It may be desired to find all of an expected set. However, in an embodiment it may be sufficient to find only a selected defined set or predefined amount, which may be less than all. Once the processing finishes, the final decoding can be done over all stored data.

The shown solution at least has the effect that the processing will be ended as soon as all desired portions of information have been stored. This avoids continuing the processing until all available oligos have been fetched or sequenced. Hence, the decoding speed is increased significantly. Further, the amount of required memory is reduced, as it is not necessary anymore to store a large amount of decoded oligos for the same ID. Furthermore, as it is not necessary to process all oligos, required processing power can be reduced.

In one embodiment the defined set of different identifiers contains less identifiers than the set of expected identifiers. Here, the processing stops before correct sequenced oligos corresponding to all of the expected set of identifiers have been fetched. This further shortens the overall processing time and may be applied, for example, depending on the overall structure of the encoded information. For example, a certain amount of quality degradation may be acceptable, e.g., in a video or audio file depending on the used encoding scheme, or the encoded overall data may comprise enough redundancy or may be encoded with an error correction code capable of reconstructing one or more missing portions of information. Further, the amount of memory provided for storing portions of information read from sequenced oligos can be reduced.

In one embodiment, the portion of information encoded in the sequenced oligo, e.g. the most recently fetched sequenced oligo, is also stored if for a first time for an oligo having associated the read identifier the evaluated error detection code indicates an error and no portion of information encoded in a previously sequenced oligo having associated the read identifier has been stored before. In other words, incorrect portions of information are stored although the error detection code indicates an error. The incorrect portions are replaced once a correct portion of information is found for the corresponding identifier. The acceptance of a certain amount of incorrect portions of information shortens the processing. The amount of acceptable incorrect portions of information depends, for example, on the encoding scheme or redundancy of the encoded data.

In one embodiment, if the evaluated error detection code indicates no error, the portion of information encoded in the sequenced oligo, e.g. the most recently fetched sequenced oligo, is additionally stored without replacing an already stored portion of information encoded in a previously sequenced oligo having associated the read identifier and for which the corresponding error detection code indicates no error. This allows improving decoding accuracy, if a larger memory block can be provided. As described above, for each oligo it is checked according to its error detection result, if the data are correct or not. However, each error detection code has an undetected error probability, where an undetected error occurs when a sequenced data block is a valid code word of the error detection code, but is different from the code word (datablock) that was synthesized. The proposed storing of more than one correct portion of information corresponding to an identical identifier allows, for example, calculation of a reliability indicator for the decoded information or a calculation of the most likely correct result.

For example, stored portions of information encoded in sequenced oligos having associated the same identifier may be compared and one or a combination of more than one of the stored portions may be selected as the correct portion depending on a decision criterion, for example a majority decision. Other decision criteria may be applied. As an example, the undetected error probability can be reduced, if additional majority decisions are introduced: For example, if for a given ID the same oligo indicated to be correct was read two times, the probability that for both oligos the error detection failed and that the error pattern is identical for both oligos, is much less than the undetected error probability for a single oligo. For using majority decisions, the memory block for storing the resulting oligo portions of information such that a plurality of oligos with a given ID can be stored is enlarged and its structure may be changed such that the number of stored oligo portions of information with different states can be managed. The process of performing majority decisions, e.g. using two, three or more oligos associated with the same ID, can be adapted to a targeted error rate and/or the available memory and processing resources.

In one embodiment the portion of information encoded in the sequenced oligo, e.g. the most recently fetched sequenced oligo, is stored if the evaluated error detection code indicates an error, at least one portion of information encoded in a previously sequenced oligo has already been stored for which the corresponding error detection code indicates an error and no portion of information encoded in a previously sequenced oligo has already been stored for which the corresponding error detection code indicates no error. More than one found incorrect portions of information for a same ID are stored, if no correct portion of information is found meanwhile. As an example, a majority decision may be applied or an error correcting or error reducing algorithm may be used. For example, by checking a plurality of these incorrect oligos it might be possible to merge at least some of them to a correct oligo or a resulting oligo with a reduced number of errors, thereby improving reliability of the result and improving overall decoding speed.

While not explicitly described, the present embodiments may be employed in any combination or sub-combination.

### BRIEF DESCRIPTION OF THE DRAWINGS

- Fig. 1: schematically illustrates a method for controlling a decoding of portions of information encoded in a plurality of synthesized oligos according to an embodiment of the invention;
- Fig. 2: schematically illustrates a method for controlling a decoding of portions of information encoded in a plurality of synthesized oligos according to another embodiment of the invention; and
- Fig. 3: schematically illustrates an apparatus for controlling a decoding of portions of information encoded in a plurality of synthesized oligos according to an embodiment of the invention.

### DETAILED DESCRIPTION OF PREFERED EMBODIMENTS

For a better understanding, the invention will now be explained in more detail in the following description with reference to the drawings. It is understood that the invention is not limited to these exemplary embodiments and that specified features can also expediently be combined and/or modified without departing from the scope of the present invention as defined in the appended claims.

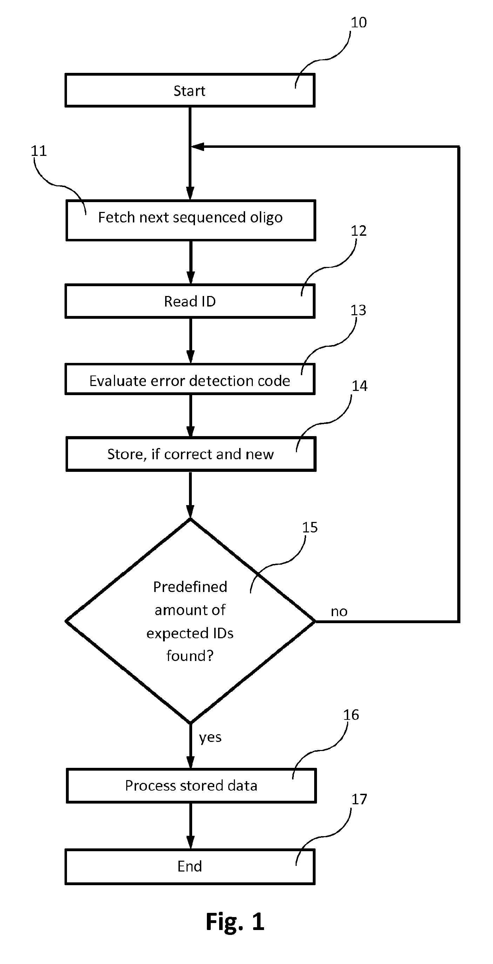

Referring to Fig.1, a method for controlling a decoding of portions of information encoded in a plurality of synthesized oligos according to an embodiment of the invention is schematically illustrated. In the following description of the figure an "oligo" refers to a nucleic acid fragment, e.g., a DNA fragment or an RNA fragment. The oligos may be available, for example, in a nucleic acid storage, e.g., of a nucleic acid storage system. A "portion of information" refers to a share of the overall information encoded in digital user (or source) data, i.e., the particular digital data encoded in a single oligo.

The synthesized oligos are encoded using error detection code and identified by associated identifiers (ID). An "error detection code" refers to any coding capable to enable detection of at least one error in the encoded portion of information. As an example, an error detection code may be a cyclic redundancy check (CRC) code. Any error correction code is regarded as a special case of an error detection code. If no error is indicated, the encoded portion of information is regarded free from symbol errors and identical to the original information. The associated identifier may or may not be protected by the error detection code.

After start 10 of the processing, in a first processing step 11, a sequenced oligo of the plurality of synthesized oligos is fetched. The plurality may comprise the originally synthesized oligos and, if applicable, their copies generated by amplification. "Fetching a sequenced oligo" comprises receiving a result of a sequencing operation performed on a synthesized oligo comprising analyzing and reading the encoded portion of information, i.e., making available the encoded digital data for storing in a memory device such as a random-access memory (RAM) or any other storage medium. In one embodiment fetching the sequenced oligo comprises sequencing the synthesized oligo, whereas in another embodiment fetching the sequenced oligo refers to fetching the result of a sequencing performed on the synthesized oligo prior to the fetching. The read information to be stored may, for example, be stored in a memory block organized in the form of a table.

In a second processing step 12, the identifier associated with the sequenced oligo is read.

In a third processing step 13, the error detection code of the sequenced oligo is evaluated. The third processing step 13 may be carried out after, simultaneously with or before the second processing step 12. For example, it may be carried out before the second processing step 12, if the identifier is protected by the error detection code, too. Otherwise, it may be carried out afterwards.

In a fourth processing step 14, the portion of information encoded in the sequenced oligo is stored, for example together with the read associated identifier, if for a first time for an oligo having associated the read identifier the evaluated error detection code indicates no error. "Storing the portion of information" refers to storing the encoded data, for example in a memory block in a RAM, a hard disk or any other storage device. This may, for example, also comprise storing a pointer, address or other identification of a location of the portion of information. In an embodiment it may refer, additionally or instead, to storing the sequenced oligo or an oligo part containing the portion of information, or a pointer, address or other identification of a location of the oligo or a part of the oligo carrying the portion of information.

The processing steps of fetching 11, reading 12, evaluating 13 and storing 14 are repeated 15 until, for at least a defined set of different identifiers of an expected set of identifiers, portions of information encoded in corresponding sequenced oligos for which the evaluated error detection code indicates no error have been stored. The amount of different identifiers may be at least one and at most the amount of expected identifiers. In other words, the processing loop is repeated until all of an expected set of identifiers have been found and corresponding portions of information have been stored or is ended before if a defined set of different identifiers has been found and corresponding portions of information have been stored, i.e. if a defined amount or a selected set of missing data is considered acceptable.

The stored information can be further processed 16 after the processing loop is ended. For example, source encoded data may be decoded and/or, depending on the encoding scheme, for example, additional error correction may be performed, before the processing ends 17.

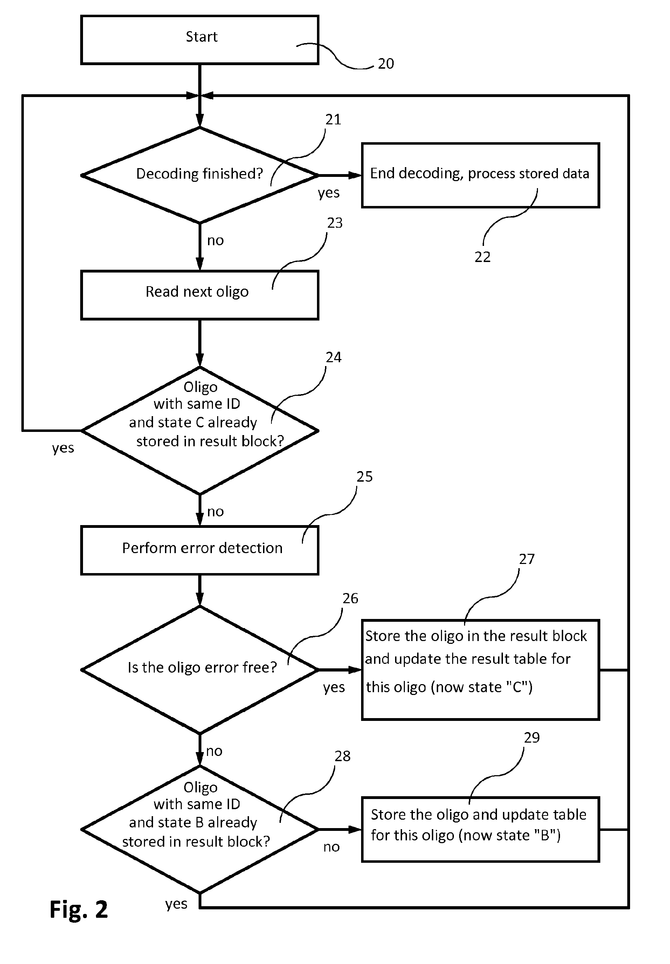

Referring to Fig. 2, a method for controlling a decoding of portions of information encoded in a plurality of synthesized oligos according to another embodiment of the invention is schematically illustrated.

The data to be decoded is provided in a plurality of oligos, wherein a given number of oligos (NO) with identifiers, e.g. identification numbers or addresses (which may or may not be contiguous), was synthesized and amplified, where each oligo has a length of LO nucleotides. Additional nucleotides forming a primer may be added at the beginning and the end of the nucleotide sequence. Each oligo contains some error detection code, i.e., at least the user data or source data is protected by the error detection code, which allows checking (with a small remaining failure rate), whether the data stored in the oligo are correct.

For decoding, memory of the size NO x LO is reserved (for example on a harddisk or in RAM etc.) as one large file or block ("result block"). In addition, a state table is created, which, for each of the NO oligos, contains the decoding state ("result table"). Result block and result table may be provided combined in a single device or separately.

Possible states to be stored in the result table for each corresponding identifier are:
"State A": No sequenced oligo with the given ID has yet been found.
"State B": A sequenced oligo with the given ID has been found, but an evaluation of the error detection code indicated an error. The oligo still contains errors.
"State C": A sequenced oligo with the given ID has been found and correctness has been confirmed by evaluation of the error detection code.

The available sequenced data are analyzed within a loop, consisting of the following steps:
After the loop start 20 it is checked in a first step 21 if the decoding is finished. If not, the processing is continued with the second step 23. Otherwise, the processing is ended 22, which may, for example, comprise further processing of the stored information.

In the second step 23 the next sequenced oligo is read.

In a third step 24 the ID is determined and the state for this ID being stored in the result table is checked. If a correct oligo with the given ID was already found before (State C), the processing continues with the first step 21. In all other cases the processing continues with the fourth step 25.

In the fourth step 25 error detection is performed for the read oligo and it is checked whether it is error free or not.

In a fifth step 27 the oligo is stored at the corresponding location in the result block and the result table for this ID is updated such that the state is changed to "State C", if the oligo is error free 26. The processing is then continued with the first step 21.

In a sixth step 29 the oligo is stored at the corresponding location in the result block and the result table for this address is updated such that the state is changed to "State B", if no sequenced oligo with the given ID was stored before 28 (State A).

In a seventh step the processing continues with the first step 21.

The second step 23 of reading a next sequenced oligo may comprise sequencing of the oligo.

In an embodiment, counters for the number of oligos having the different states can be used, which are updated whenever the content in the result table is modified. This allows checking easily whether the decoding can be completed in processing step 1: If the number of "State C" entries is equal to the table length (NO), the decoding is finished.

Further, the number of required oligos with state C can be reduced, e.g., to 0.999 x NO, if a low error rate can be accepted or if additional error correction code is used that is able to recover the missing oligos.

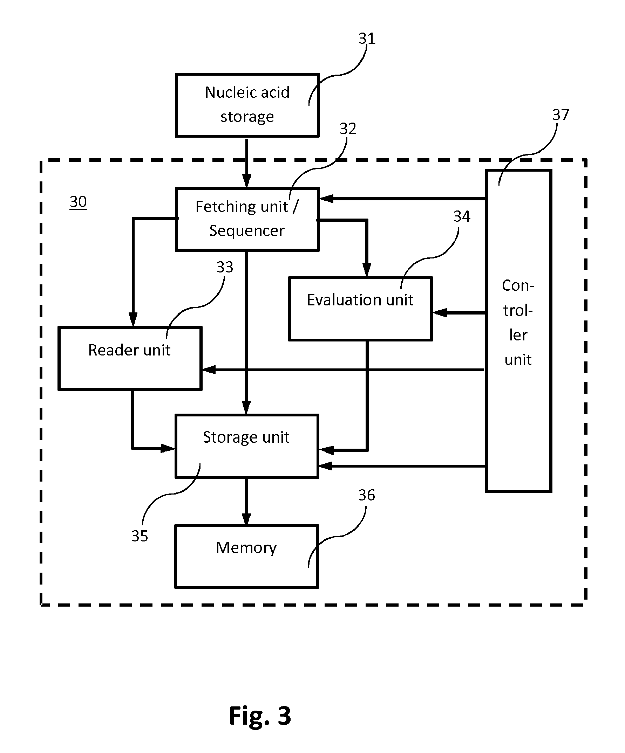

Referring to Fig. 3, an apparatus 30 for controlling a decoding of portions of information encoded in a plurality of synthesized oligos according to an embodiment of the invention is schematically illustrated. The apparatus 30 may, for example, be a part of a nucleic acid storage system, such as a DNA storage system. The described apparatus allows implementing the advantages and characteristics of the described method as part of the apparatus for controlling a decoding of portions of information encoded in a plurality of synthesized oligos.

Each of the oligos to be decoded is encoded using an error detection code and identified by an associated identifier. The apparatus 30 comprises a fetching unit 32, a reader unit 33, an evaluation unit 34, a storage unit 35, a memory 36 and a controller unit 37.

The fetching unit 32 contains or is connectable to a nucleic acid storage 31 which at least contains the plurality of synthesized oligos, which may include amplified oligos, i.e., multiple copies of identical oligos. The synthesized oligos may be stored, for example, either as solid matter or dissolved in a liquid, in the nucleic acid storage 31 as a container. The characteristics of the nucleic acid storage 31 may depend on the amount of stored data and an expected time before a readout of the data will take place.

The fetching unit 32 may, for example, comprise a sequencer or be connected or connectable to a sequencer unit which is connected or connectable to other units of the apparatus via an interface through which read nucleotide sequences are accessible. The fetching unit 32 is configured to fetch sequenced oligos of the plurality of synthesized oligos. The fetching unit 32 which, for example, either receives the stored nucleic acid fragments or oligos from the nucleic acid storage 31, determines the sequences of nucleotides of the particular oligo and provides read code symbols corresponding to the read sequences of nucleotides to the connected units, or receives the sequencing results from a sequencer unit. In Fig. 3, block 32 illustrates a fetching unit which comprises the sequencer. In another embodiment, the sequencer may be a separate unit between the nucleic acid storage and the fetching unit 32.

The reader unit 33 is connected to the fetching unit 32 and is configured to read the identifiers associated with the sequenced oligos.

The evaluation unit 34 is connected to the fetching unit 32 and is configured to evaluate the error detection codes of the portions of information encoded in the sequenced oligos.

The storage unit 35 is connected to the fetching unit 32, the reader unit 33 and the evaluation unit 34. The storage unit 35 is configured to store portions of information encoded in sequenced oligos, if for a first time for an oligo having associated a read identifier the evaluated error detection code indicates no error. The storage unit 35 shown in Fig.3 implements the functionality of receiving and storing the read portions of information or complete read nucleotide sequences, the corresponding identifiers and error evaluation results in a memory device 36, such as a RAM or other memory circuitry, hard disk etc. connected to the storage unit 35. However, in an embodiment the storage unit 35 may contain the memory device 36 instead of being connected to it.

The controller unit 37 is connected to the fetching unit 32, the reader unit 33, the evaluation unit 34 and the storage unit 35. It is configured to repeatedly actuate the fetching unit 32, the reader unit 33, the evaluation unit 34 and the storage unit 35 until, for at least a defined set of different identifiers of an expected set of identifiers, portions of information encoded in corresponding sequenced oligos for which the evaluated error detection code indicates no error have been stored. The term actuate, i.e., activate or switch on, refers to any operation to timely trigger, enable or put a particular unit of the apparatus 30 into a state to perform the desired action.

The reader unit 33, the evaluation unit 34, the storage unit 35 and the controller unit 37 may, for example, be provided as separate devices, jointly as at least one device or logic circuitry, or functionality carried out by a microprocessor, microcontroller or other processing device, computer or other programmable apparatus.

The apparatus 30 may provide further functionality to process the portions of information stored in the memory 36, for example implemented in an additional processing device, information decoder unit or the controller unit 37.

The proposed solution uses an error detection code to check, whether an oligo contains correct information. Only correct information is processed in a specific way such that the final decoding can be done over all stored data while sequencing or, respectively, fetching of sequencing results is carried out only for a few of all synthesized and amplified oligos. The decoding speed is improved significantly and the required memory and processing power is reduced. With this solution, retrieval of information stored in nucleic acid storages is improved.

As will be appreciated by one skilled in the art, aspects of the present principles can be embodied as an apparatus, method or computer readable medium. Accordingly, aspects of the present principles can take the form of a hardware embodiment, a software embodiment or an embodiment combining software and hardware aspects. Furthermore, aspects of the present principles can take the form of a computer readable storage medium. Any combination of one or more computer readable storage medium(s) may be utilized.

Apart from the bio-chemical processing when sequencing oligos, aspects of the invention may, for example, at least partly be implemented in a computer program comprising code portions for performing steps of the method according to an embodiment of the invention when run on a programmable apparatus or enabling a programmable apparatus to perform functions of an apparatus according to an embodiment of the invention.

Any connection shown may be a direct connection or an indirect connection. Further, those skilled in the art will recognize that the boundaries between logic blocks are merely illustrative and that alternative embodiments may merge logic blocks or impose an alternate decomposition of functionality upon various logic blocks.

## Claims

1. A method for controlling a decoding of portions of information encoded in a plurality of synthesized oligos, encoded using an error detection code and identified by an associated identifier, the method comprising:
- fetching (11) a sequenced oligo of the plurality of synthesized oligos;
- reading (12) the identifier associated with the sequenced oligo;
- evaluating (13) the error detection code of the sequenced oligo;
- storing (14) the portion of information encoded in the sequenced oligo, if for a first time for an oligo having associated the read identifier the evaluated error detection code indicates no error; and
- repeating (15) the fetching (11), reading (12), evaluating (13) and storing (14) until, for at least a defined set of different identifiers of an expected set of identifiers, portions of information encoded in corresponding sequenced oligos for which the evaluated error detection code indicates no error have been stored.

2. The method according to claim 1, wherein the defined set of different identifiers contains less identifiers than the set of expected identifiers.

3. The method according to claim 1 or claim 2, wherein the portion of information encoded in the sequenced oligo is also stored if for a first time for an oligo having associated the read identifier the evaluated error detection code indicates an error and no portion of information encoded in a previously sequenced oligo having associated the read identifier has been stored before.

4. The method according to one of the preceding claims, wherein, if the evaluated error detection code indicates no error, the portion of information encoded in the sequenced oligo is additionally stored without replacing an already stored portion of information encoded in a previously sequenced oligo having associated the read identifier and for which the corresponding error detection code indicates no error.

5. The method according to claim 4, further comprising
- comparing stored portions of information encoded in sequenced oligos having associated the same identifier and selecting one or a combination of more than one of the stored portions as the correct portion depending on a decision criterion.

6. The method according to claim 3, wherein the portion of information encoded in the sequenced oligo is also stored if the evaluated error detection code indicates an error, at least one portion of information encoded in a previously sequenced oligo has already been stored for which the corresponding error detection code indicates an error and no portion of information encoded in a previously sequenced oligo has already been stored for which the corresponding error detection code indicates no error.

7. An apparatus (30) for controlling a decoding of portions of information encoded in a plurality of synthesized oligos, encoded using an error detection code and identified by an associated identifier, the apparatus (30) comprising:
- a fetching unit (32) configured to fetch sequenced oligos of the plurality of synthesized oligos;
- a reader unit (33), connected to the fetching unit (32) and configured to read the identifiers associated with the sequenced oligos;
- an evaluation unit (34), connected to the fetching unit (32) and configured to evaluate the error detection codes of the portions of information encoded in the sequenced oligos;
- a storage unit (35), connected to the fetching unit (32), the reader unit (33) and the evaluation unit (34), and configured to store portions of information encoded in sequenced oligos, if for a first time for an oligo having associated a read identifier the evaluated error detection code indicates no error; and
- a controller unit (37), connected to the fetching unit (32), the reader unit (33), the evaluation unit (34) and the storage unit (35), and configured to repeatedly actuate the fetching unit (32), the reader unit (33), the evaluation unit (34) and the storage unit (35) until, for at least a defined set of different identifiers of an expected set of identifiers, portions of information encoded in corresponding sequenced oligos for which the evaluated error detection code indicates no error have been stored.

8. The apparatus (30) according to claim 7, wherein the controller unit (37) is configured to set the amount of different identifiers in the defined set of different identifiers less than the amount of identifiers contained in the set of expected identifiers.

9. The apparatus (30) according to claim 7 or claim 8, wherein the storage unit (35) is further configured to store the portion of information encoded in the sequenced oligo, if for a first time for an oligo having associated the read identifier the evaluated error detection code indicates an error and no portion of information encoded in a previously sequenced oligo having associated the read identifier has been stored before.

10. The apparatus (30) according to one of claims 7 to 9, wherein the storage unit (35) is further is configured to additionally store the portion of information encoded in the sequenced oligo if the evaluated error detection code indicates no error without replacing an already stored portion of information encoded in a previously sequenced oligo having associated the read identifier and for which the corresponding error detection code indicates no error.

11. The apparatus (30) according to claim 10, wherein the storage unit (35) is further configured to compare stored portions of information encoded in sequenced oligos having associated the same identifier and to select one or a combination of more than one of the stored portions as the correct portion depending on a decision criterion.

12. The apparatus (30) according to claim 9, wherein the storage unit (35) is further configured to store the portion of information encoded in the sequenced oligo if the evaluated error detection code indicates an error, at least one portion of information encoded in a previously sequenced oligo has already been stored for which the corresponding error detection code indicates an error and no portion of information encoded in a previously sequenced oligo has already been stored for which the corresponding error detection code indicates no error.

13. A computer readable storage medium having stored therein instructions enabling control of a decoding of portions of information encoded in a plurality of synthesized oligos, encoded using an error detection code and identified by an associated identifier, which, when executed by a computer, cause the computer to:
- actuate a fetching unit connectable to the computer to fetch (11) a sequenced oligo of the plurality of synthesized oligos;
- read (12) the identifier associated with the sequenced oligo;
- evaluate (13) the error detection code of the sequenced oligo;
- store (14) the portion of information encoded in the sequenced oligo, if for a first time for an oligo having associated the read identifier the evaluated error detection code indicates no error; and
- repeat (15) the actuating the fetching (11), the reading (12), the evaluating (13) and the storing (14) until, for at least a defined set of different identifiers of an expected set of identifiers, portions of information encoded in corresponding sequenced oligos for which the evaluated error detection code indicates no error have been stored.

14. The computer readable storage medium according to claim 13, further having stored therein instructions which, when executed by the computer, cause the computer to store the portion of information encoded in the sequenced oligo, if for a first time for an oligo having associated the read identifier the evaluated error detection code indicates an error and no portion of information encoded in a previously sequenced oligo having associated the read identifier has been stored before.

15. The computer readable storage medium according to claim 13 or claim 14, further having stored therein instructions which, when executed by the computer, cause the computer to additionally store the portion of information encoded in the sequenced oligo if the evaluated error detection code indicates no error without replacing an already stored portion of information encoded in a previously sequenced oligo having associated the read identifier and for which the corresponding error detection code indicates no error.
